# EUROPEAN PATENT APPLICATION

(11) **EP 3 756 645 A1**
(43) Date of publication of application: **30.12.2020**
(21) Application number: 19780130.1
(22) Date of filing: 30.05.2019
(51) Int. Cl.: A61K 8/42, A61K 8/06, A61K 8/23, A61K 8/24, A61K 8/36, A61K 8/362, A61K 8/365, A61K 8/44, A61K 8/49, A61Q 5/06, A61Q 5/12

(54) **HAIR COSMETIC**

(30) Priority: 14.05.2019 JP 2019091486
(71) Applicant: Kokyu Alcohol Kogyo Co., Ltd., Narita-shi Chiba 287-0225 (JP)
(72) Inventor: The designation of the inventor has not yet been filed
(74) Representative: Cabinet Nuss
(86) International application number: PCT/JP2019/021492
(87) International publication number: WO 2020/230337

(57) **Abstract**

An object is to provide a hair cosmetic that has high hair dyeing property and that provides good smoothness for hair.

An emulsion type hair cosmetic comprising
an amide alcohol represented by formula (I) wherein R₁ is an optionally substituted C6-C22 hydrocarbon group, R₂ is H, or an optionally substituted C6-C22 hydrocarbon group, and R₃ is an optionally substituted, linear or branched C2-C21 hydrocarbon group; an organic acid; and any one dye of an HC dye, a basic dye and/or an acid dye.

## Description

### [Technical Field]

The present invention relates to an emulsion type hair cosmetic comprising an amide alcohol.

### [Background Art]

Conventionally, as a hair coloring product, a hair dyeing agent such as a hair color that is a permanent hair dye agent classified as a quasi-drug and a hair dyeing material such as a hair manicure and a color treatment that is a semi-permanent hair dyeing agent having little or no damage to the hair and that is classified as a cosmetic, and the like are used. In general, a hair color is a hair dyeing agent that uses an oxidative reaction, and it tends to cause skin irritation while its color is lasting, and thus skin allergy test (patch test) is required when it is used, and the damage to the hair is inevitable. On the other hand, a hair manicure and a color treatment have the characteristics that they do not cause skin irritation and cause little damage to the hair, although their color lasting is inferior to that of a hair color. Furthermore, in the conventional coloring products, various cationic surfactants are used in order to enhance the hair feel effect (Patent literatures 1 to 4), however, there was a problem that cationic surfactants cause skin irritation and have poor biodegradability and thus cause adverse effects on the environment.

On the other hand, in cosmetics, it is known that an amide alcohol can be used as an oil gelling agent in a hair care product (Patent literature 5), however it has not been known that it is used in an emulsion type hair cosmetic for dyeing hair such as a color treatment.

Therefore, an object of the present invention is to provide a hair cosmetic that does not use a surfactant, has high hair dyeing properties, and provides good smoothness (ease of slipping) .

### [Prior Art Literatures]

### [Patent Literatures]

[Patent literature 1] JP 2016-193872 A
[Patent literature 2] JP 2019-19097 A
[Patent literature 3] JP 2017-160198 A
[Patent literature 4] JP 2017-31086 A
[Patent literature 5] JP 2015-67571 A

### [Summary of the Invention]

### [Problems to Be Solved by the Invention]

In view of the above-described problems in the prior art, an object of the present invention is to provide a hair cosmetic that has high hair dyeing properties and provides good smoothness (ease of slipping) for hair. In particular, an object is to provide a hair cosmetic having excellent hair dyeing properties for hair.

### [Means for Solving the Problems]

In order to solve the above-mentioned problems, during diligent researches, the present inventors have found that specific amide alcohols enhance the hair dyeing properties of a hair cosmetic, and as a result of further studies, they have completed the present invention.

The present invention relates to the followings:
[1] An emulsion type hair cosmetic comprising
   an amide alcohol represented by formula (I) 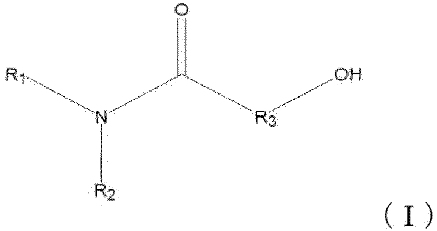 wherein
   R₁ is an optionally substituted C6-C22 hydrocarbon group,
   R₂ is H, or an optionally substituted C6-C22 hydrocarbon group,
   R₃ is an optionally substituted, linear or branched C2-C21
   hydrocarbon group,
   an organic acid, and
   any one dye of an HC dye, a basic dye and/or acid dye.
[2] The emulsion type hair cosmetic according to [1] comprising the amide alcohol represented by formula (I) wherein R₁ is C10-C22 hydrocarbon group,
   R₂ is H,
   R₃ is C3-C12 hydrocarbon group.
[3] The emulsion type hair cosmetic according to [1] or [2], wherein the amide alcohol is 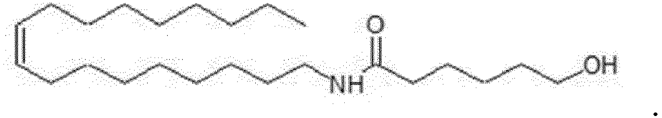
[4] The emulsion type hair cosmetic according to any one of [1] to [3] comprising 0.1 to 10.0% by mass of the amide alcohol represented by formula (I),
   0.1 to 1.0% by mass of the organic acid, and
   0.1 to 1.0% by mass of any one dye of an HC dye, a basic dye and/or acid dye.
[5] The emulsion type hair cosmetic according to any one of [1] to [4], wherein the molar ratio of the amide alcohol represented by formula (I) and the organic acid is 3:1 to 9:1.
[6] The emulsion type hair cosmetic according to any one of [1] to [5], wherein the emulsion type hair cosmetic is used for dyeing hair.
[7] The emulsion type hair cosmetic according to any one of [1] to [6], wherein the organic acid is one or more selected from the group consisting of monocarboxylic acid, dicarboxylic acid, hydroxycarboxylic acid, polycarboxylic acid, acidic amino acid and fatty acid.
[8] The emulsion type hair cosmetic according to any one of [1] to [7], wherein the organic acid is selected from glutamic acid, aspartic acid, lactic acid, citric acid, pyrrolidone carboxylic acid, malic acid, glycolic acid, formic acid, oxalic acid, benzoic acid, phosphoric acid, and sulfonic acid.
[9] The emulsion type hair cosmetic according to any one of [1] to [8] comprising one or more oil agents selected from the group consisting of an ester oil, a silicone oil, and a hydrocarbon oil.
[10] The emulsion type hair cosmetic according to any one of [1] to [9] which is substantially free of surfactants.
[11] The emulsion type hair cosmetic according to any one of [1] to [10] comprising glutamic acid as the organic acid.
[12] The emulsion type hair cosmetic according to any one of [9] to [11] comprising squalene, liquid paraffin and/or a non-volatile hydrocarbon oil as the oil agent.

### [Advantageous Effects of the Invention]

The present invention provides a hair cosmetic that has a high hair dyeing property and gives a good smoothness to the hair by using a specific amide alcohol.

In particular, the effect becomes remarkable by using the HC dye or basic dye of the present invention.

### [Brief Description of Drawings]

[FIG. 1] Fig. 1 is a figure showing emulsifying property when each dye is used in the presence of carbomer.
[FIG. 2] Fig. 2 is a figure showing the difference in hair dyeing properties when the amide alcohol is used and when a surfactant is used.
[FIG. 3] Fig. 3 is a figure showing the emulsified state of each blended amount of the amide alcohol.
[FIG. 4] Fig. 4 is a figure showing the difference in smoothness of each blended amount of the amide alcohol.
[FIG. 5] Fig. 5 is a figure showing hair dyeing properties when the HC dye are used and when basic dye are used, in the case of using the amide alcohol or a surfactant.
[FIG. 6] Fig. 6 is a figure showing smoothness (ease of slipping) when the HC dye are used and when basic dye are used, in the case of using the amide alcohol or a surfactant.
[FIG. 7] Fig. 7 is a figure showing emulsifying property when the basic dye is used.
[FIG. 8] Fig. 8 is a figure showing emulsifying property when the acid dye is used.
[FIG. 9] Fig. 9 is a figure showing comparison in dyeing properties when the amide alcohol is used and when a surfactant is used.
[FIG. 10] Fig. 10 is a figure showing the measurement result of positive ions of AMINACTOL OLH by TOF-SIMS.
[FIG. 11] Fig. 11 is a figure showing the measurement result of negative ions of AMINACTOL OLH by TOF-SIMS.
[FIG. 12] Fig. 12 is a figure showing the measurement result of positive ions of Genamin STAC by TOF-SIMS.
[FIG. 13] Fig. 13 is a figure showing the measurement result of negative ions of Genamin STAC by TOF-SIMS.
[FIG. 14] Fig. 14 is a figure showing the measurement result of positive ions of damaged hair by TOF-SIMS.
[FIG. 15] Fig. 15 is a figure showing the measurement result of negative ions of damaged hair by TOF-SIMS.
[FIG. 16] Fig. 16 is a figure showing the measurement result of positive ions of damaged hair treated with AMINACTOL OLH by TOF-SIMS.
[FIG. 17] Fig. 17 is a figure showing the measurement result of negative ions of damaged hair treated with AMINACTOL OLH by TOF-SIMS.
[FIG. 18] Fig. 18 is a figure showing the measurement result of positive ions of damaged hair treated with Genamin STAC by TOF-SIMS.
[FIG. 19] Fig. 19 is a figure showing the measurement result of negative ions of damaged hair treated with GenaminSTAC by TOF-SIMS.
[FIG. 20] Fig. 20 is a figure showing mapping image by TOF-SIMS.

### [Description of Embodiments]

### Component (A): Amide alcohol

The amide alcohol used in the present invention is represented by the following formula (I): 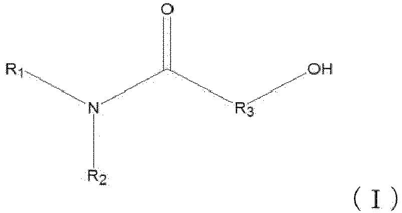 wherein
R₁ is an optionally substituted C6-C22 hydrocarbon group, R₂ is H, or an optionally substituted C6-C22 hydrocarbon group, R₃ is an optionally substituted, linear or branched C2-C21 hydrocarbon group.

As used herein, the term "hydrocarbon group" may be, unless otherwise specified, saturated or unsaturated, linear, branched or cyclic, or a combination of linear or branched with cyclic, and includes, for example, a hydrocarbon group consisting of a linear or branched hydrocarbon moiety and a cyclic hydrocarbon moiety such as benzyl group, phenylethyl group, etc..

That is, the C6-C22 hydrocarbon group in R₁ and R₂ includes a linear, branched or cyclic C6-C22 hydrocarbon group, or a C6-C22 hydrocarbon group consisting of a linear or branched hydrocarbon moiety and a cyclic hydrocarbon moiety, and examples thereof include cyclic groups such as cyclohexyl, decahydronaphthyl, tetrahydrodicyclopentadiene, sterol, phenyl, naphthyl, anthracenyl, etc.; branched alkyl groups such as ethylhexyl, isostearyl, octyldodecyl, etc.; multibranched alkyl groups such as dimethyl, trimethyl , tetramethyl, etc.; linear alkyl groups such as hexyl, octyl, lauryl, myristyl, cetyl, stearyl, arachyl, behenyl, etc.; and alkenyl groups such as oleyl and elaidyl, etc.

In one embodiment of the present invention, R₁ is preferably an unsubstituted hydrocarbon group. Specifically, it is preferably an unsaturated linear or branched C10-C22 hydrocarbon group; or a cyclic C6-C22 hydrocarbon group; or a benzyl group or a phenylethyl group.

The specific examples of R₁ include cyclohexyl, ethylhexyl, octyl, lauryl, myristyl, stearyl, oleyl, benzyl or phenylethyl.

In one embodiment of the present invention, R₁ is particularly preferably lauryl, myristyl, stearyl and oleyl.

In one embodiment of the present invention, R₂ is preferably H.

The hydrocarbon group in R₃ is a linear or branched C2-C21 hydrocarbon group having no cyclic structure, and examples thereof include alkyl groups such as propyl, butyl, pentyl, hexyl, heptyl, octyl, ethylhexyl, etc., and alkenyl groups such as butylene, pentylene, hexylene, heptylene, etc.

In one embodiment of the present invention, R₃ is preferably a linear or branched C2-C12 hydrocarbon group, preferably an alkyl group, and specifically, propylene, butylene, pentylene or hexylene.

In the present invention, each hydrocarbon group may be substituted, and may be substituted with, for example, a hydroxy group, a carboxy group, an aldehyde group.

Examples of substituted C6-C22 hydrocarbon group in R₁ and R₂ include hexanol, ethylcyclohexanol, hexanoic acid.

Examples of substituted C2-C21 hydrocarbon group in R₃ include hydroxybutyl, butyl ketone.

In one embodiment of the present invention, the amide alcohol of formula (I) wherein R₁ is C10-C22 hydrocarbon group, R₂ is H, and R₃ is C3-C12 hydrocarbon group is preferable, and the amide alcohol of formula (I) wherein R₁ is C12-C18 hydrocarbon group, R₂ is H, and R₃ is C3-C5 hydrocarbon group is particularly preferable.

In a preferred embodiment of the present invention, the amide alcohol of formula (I) is N-oleyl-6-hydroxyhexyl amide (Cosmetic Ingredient Name: N-oleyl hydroxyhexanamide, also referred to herein as AMINACTOL OLH) having the following structure

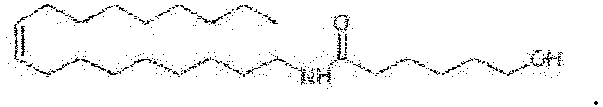

Amide alcohols can be prepared using known synthetic methods. Examples include aminolysis reaction of acid chloride and amine (Schotten-Baumann reaction), aminolysis reaction of fatty acid anhydride and amine, aminolysis reaction of methyl ester and amine, aminolysis reaction of fatty acid and amine, aminolysis reaction of lactone and amine, and the like.

Specifically, for example, it can be synthesized by the method described in Japanese Patent Application No. 2016-114276.

The amide alcohol used in the present invention is not limited, but N-oleyl-6-hydroxyhexylamide (AMINACTOL OLH), N-stearyl-4-hydroxybutyramide, N-lauryl-6-hydroxyhexylamide, N-stearyl-6-hydroxyhexanamide, alkyloylpropyl-6-hydroxyhexanamide alkyloylpropyl, N-octyl-6-hydroxyhexylamide, N-coconut fatty acid-6-hydroxyhexylamide, N-2-ethylhexyl-4-hydroxybutyramide, N-octyl-4-hydroxybutyramide, N-lauryl-4-hydroxybutyramide, N-myristyl-4-hydroxybutyramide, N-oleyl-4-hydroxybutyramide and the like can be used, and it is preferably N-oleyl-6-hydroxyhexylamide, N-stearyl-4-hydroxybutyramide, N-lauryl-6-hydroxyhexylamide, N-stearyl-6-hydroxyhexanamide, alkyloylpropyl-6-hydroxyhexanamide alkyloylpropyl, N-octyl-6-hydroxyhexylamide, N-coconut fatty acid-6-hydroxyhexylamide, and particularly preferably N-oleyl-6-hydroxyhexylamide.

The hair cosmetic in the present specification comprises the amide alcohol represented by formula (I), and may be a hair cosmetic for coloring (dyeing) hair, and may be a hair color that is a permanent hair dyeing agent, a color treatment or a hair manicure that is a semi-permanent hair dyeing agent, or a hair mascara or a hair foundation that is a temporary hair dyeing material, and the like, and it is preferably a hair dyeing material classified as a semi-permanent dyeing agent.

For a hair manicure, an acid dye may be used. Since an acid dye has a negative ion, the hair is dyed by binding to positively charged hair with ionic bonds.

For a color treatment, an acid dye, a basic dye or an HC dye may be used, and a basic dye or an HC dye is preferably used. Since a basic dye has a positive ion, it is easily adsorbed to the damaged hair that is negatively charged.

The hair cosmetic in the present invention comprises the amide alcohol represented by formula (I), the organic acid, and any one dye of an HC dye, a basic dye and/or acid dye.

In the present invention, high dyeing property and smoothness can be provided to a hair cosmetic by using the amide alcohol represented by formula (I).

In one embodiment of the present invention, the blended amount of the amide alcohol represented by formula (I) in the hair cosmetic is not particularly limited, but from the viewpoint of providing sufficient hair dyeing and smoothness effects, it may be 0.5 to 15.0% by mass, preferably 1.0 to 12.0% by mass, more preferably 3.0 to 10.0% by mass.

### Component (B): Organic acid

In the present invention, the hair cosmetic can be emulsified by using an organic acid. In one embodiment of the present invention, the pH can be adjusted by using an organic acid.

The organic acid used in the present invention is not limited as long as it emulsifies due to an interaction with the amide alcohol represented by formula (I), for example, a monocarboxylic acid such as formic acid, acetic acid, propionic acid, benzoic acid; a dicarboxylic acid such as oxalic acid, malonic acid, succinic acid, glutaric acid, adipic acid, maleic acid, fumaric acid, phthalic acid; a hydroxycarboxylic acid such as glycolic acid, lactic acid, hydroxyacrylic acid, glyceric acid, malic acid, tartaric acid, citric acid; a polycarboxylic acid such as polyglutamic acid; an acidic amino acid such as glutamic acid and aspartic acid; a fatty acid such as lauric acid, myristic acid, palmitic acid, stearic acid, isostearic acid, oleic acid, erucic acid, behenic acid, dimer acid, hydroxystearic acid, castor oil fatty acid; phosphoric acid, sulfonic acid, pyrrolidone carboxylic acid can be used; preferably glutamic acid, aspartic acid, lactic acid, citric acid, pyrrolidone carboxylic acid, malic acid, formic acid, oxalic acid, benzoic acid, phosphoric acid, sulfonic acid, and pyrrolidone carboxylic acid can be used, and particularly preferably glutamic acid can be used. In the present invention, these organic acids can be used alone or in combination of two or more.

In one embodiment of the present invention, the blended amount of the organic acid in the hair cosmetic is not particularly limited, but from the viewpoint of pH adjustment, it may be 0.1 to 10.0% by mass, preferably 0.1 to 5.0% by mass, more preferably 0.1 to 3.0% by mass.

In one embodiment of the present invention, the hair cosmetic is an O/W type emulsion.

In one embodiment of the present invention, from the viewpoint of obtaining a stable emulsion, the molar ratio of the amide alcohol represented by formula (I) and the organic acid is preferably within a range of 30:1 to 1:1, preferably within the range of 9:1 to 3:1.

### Component (C): Dye

The dye used in the present invention may be any one of an acid dye, a basic dye or an HC dye, preferably it may be a basic dye or an HC dye.

Examples of an acid dye include, but are not limited to, Black No. 401, Red No. 227, Blue No. 1, Violet No. 401, Orange No. 205, Red No. 106, Yellow No. 203, Acid Orange 3, and the like can be used.

Examples of a basic dye include, but are not limited to, Basic Red 76, Basic Red 51, Basic Blue 75, Basic Blue 99, Basic Brown 16, Basic Brown 17, Basic Yellow 57, Basic Yellow 87, Basic Orange 31 and the like can be used.

Examples of an HC dye include, but are not limited to, HC Red No. 1, HC Red No. 3, HC Red No. 7, HC Blue No. 2, HC Yellow No. 2, HC Yellow No. 4, HC Yellow No. 5, HC Orange No. 1, and the like can be used.

pH of the basic dye or the HC dye is not limited, but may be within the range of pH of 7 to 11, and preferably within the range of pH 7 to 9.

### Component (D): Oil agent

The hair cosmetic of the present invention may further comprise an oil agent. Examples of the oil agent used in the present invention include, but are not limited to, a higher alcohol such as octyldodecanol and isostearyl alcohol; an ester oil such as isotridecyl isononanoate, octyldodecyl myristate, triethylhexanoin, and diisostearyl malate; a hydrocarbon oil such as squalane, mineral oil, hydrogenated polyisobutene; a silicone oil such as dimethicone and cyclopentasiloxane, ethers such as ethylene oxide / propylene oxide dimethyl ether, and perfluoroether. Moreover, the present invention can use these oil agents alone or in combination of two or more.

In one embodiment of the present invention, from the viewpoint of obtaining a stable emulsion, the oil agent is preferably an ester oil, a silicone oil, or a hydrocarbon oil, and more preferably the oil agent may be squalene, liquid paraffin and/or a nonvolatile hydrocarbon oil.

### Component (E): Polyhydric alcohol

The hair cosmetic of the present invention may further comprise a polyhydric alcohol.

The polyhydric alcohol used in the present invention is not particularly limited as long as it is a compound having a plurality of hydroxy groups in the molecule, but from the viewpoint of providing appropriate emulsifying ability, typically it is a polyhydric alcohol with 3 to 10, preferably 5 to 8 carbon atoms. Further, the number of hydroxy groups in the polyhydric alcohol molecule is 2 to 4, preferably 2.

Specific examples of the polyhydric alcohol include, for example, pentylene glycol, 2-ethyl-1,3-hexanediol, 1,2-pentanediol, 1,3-butylene glycol, propane-1,2,3-triol, propane-1,2-diol, 1,2-octanediol, 1,2-hexanediol, and the like, and it is preferably pentylene glycol or 2-ethyl-1,3-hexanediol.

Polyhydric alcohols can be used alone or in combination of two or more.

The content of polyvalent alcohol is 0.1 to 20.0% by weight, preferably 1.0 to 15.0% by weight, and most preferably 1.0 to 10.0% by weight.

By using the polyhydric alcohol, it can be predicted that the emulsion is stabilized by hydrogen bonding with the amide alcohol and the organic acid.

### <Other components>

The hair cosmetic of the present invention may contain any components used in various cosmetics such as a hair conditioner.

Examples of these additional components include an UV absorber such as ethylhexyl methoxycinnamate and diethylamino hydroxybenzoyl hexyl benzoate; a thickener and a gelling agent such as dextrin palmitate and xanthan gum; a quality-maintaining component such as an antioxidant and a preservative; various medicinal ingredients / active ingredients; a colorant; a fragrance and the like.

In the present invention, the term "emulsion type" refers to the one in the form of an emulsion, particularly the one in the form of an O/W emulsion. The hair cosmetic of the present invention is considered to have improved hair dyeing property because it is an emulsion type.

In a special embodiment of the present invention, the hair cosmetic is an emulsion type hair cosmetic that is substantially free of surfactants such as a cationic surfactant.

In the present specification, the term a "surfactant" means a compound having both a hydrophilic group and a hydrophobic group in one molecule. Here, "substantially free of surfactants" means that it comprises no surfactant at all or comprises a surfactant in an amount that does not emulsify. The amount that does not emulsify can be appropriately determined by a person skilled in the art according to its composition, for example, in one embodiment it is less than 2.0% by mass, in another embodiment it is less than 0.2 % by mass, or less than 0.02 % by mass.

Hereinafter, the present invention will be described in more detail based on examples; however, the present invention is not limited to these examples, and various modifications can be made without departing from the technical idea of the present invention. In the present specification, unless otherwise specified, % means % by weight.

### [Example]

### Experiment 1: Comparison of emulsification in carbomer when each dye is used

As a dye, HC Red No. 3 (manufactured by JOS.H.LOWENSTEIN & SONS, INC) and Basic Red 76 (manufactured by SENSIENT Corporation) are used, and the emulsion type hair cosmetics were prepared according to the following formulation.

**Table 1. A formulation of an emulsion type hair cosmetic under the presence of Carbopol**

| | Product name | % by weight |
|---|---|---|
| Oil phase | Mineral oil | 12.0 |
| | Alcohol NO.20-B | 3.5 |
| | AMINACTOL OLH | 3.0 |
| Water phase | TRIOL VE | 5.0 |
| | DIOL PD | 3.0 |
| | HAISUGARCANE BG | 3.0 |
| | 10% Potassium hydroxide aqueous solution | 1.5 |
| | Carbopol ETD2050 | 0.3 |
| | Dye | 0.1 |
| | Purified water | 68.6 |

| | | |
|---|---|---|
| Alcohol NO.20-B: Hydrogenated rapeseed alcohol, manufactured by KOKYU ALCOHOL KOGYO CO., LTD AMINACTOL OLH: N-Oleyl hydroxyhexanamide, manufactured by KOKYU ALCOHOL KOGYO CO., LTD TRIOL VE: Glycerin, manufactured by KOKYU ALCOHOL KOGYO CO., LTD DIOL PD: Pentylene glycol, manufactured by KOKYU ALCOHOL KOGYO CO., LTD HAISUGARCANE BG: 1,3-Butylene glycol, manufactured by KOKYU ALCOHOL KOGYO CO., LTD | | |

### Preparation method

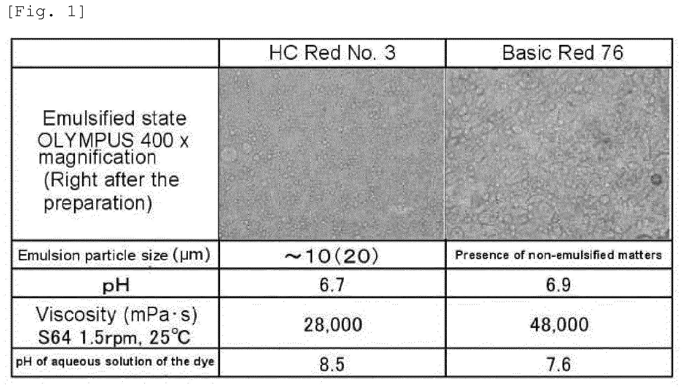

(1) Uniformly dissolve oil phase and water phase separately at 80°C
(2) Add oil phase while stirring water phase with an agitator (800rpm)
(3) After emulsification, stir with an agitator (2000rpm, 3min)
(4) Slowly cool to room temperature with manual stirring The evaluation results are shown in Fig. 1.

In the presence of carbomer, it was emulsified when an HC dye was used, but the presence of non-emulsified material was confirmed when a basic dye was used.

### Experiment 2: Comparison of emulsification and hair dyeing properties of emulsion type hair cosmetics in the presence of glutamic acid and hydroxyethyl cellulose

Using HC Red No. 3 (manufactured by JOS.H.LOWENSTEIN & SONS, INC) and Basic Red 76 (manufactured by SENSIENT Corporation) as dyes, the emulsion type hair cosmetics were prepared according to the following formulation.

As the surfactant, Genamin STAC (Steartrimonium chloride), which is a cationic surfactant, was used.

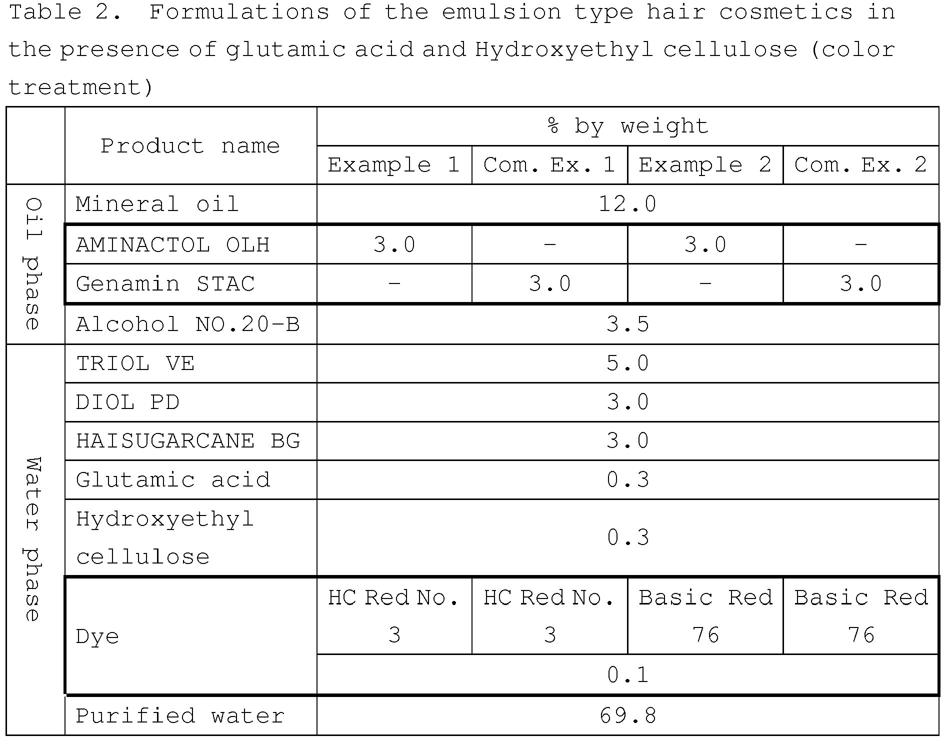

### Preparation method

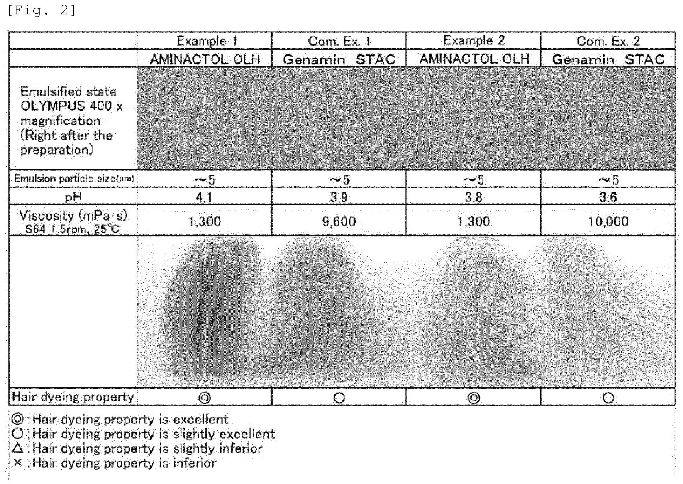

(1) Uniformly dissolve oil phase and water phase separately at 80°C
(2) Add oil phase while stirring water phase with an agitator (800rpm)
(3) After emulsification, stir with an agitator (2000rpm, 3min)
(4) Slowly cool to room temperature with manual stirring The evaluation results are shown in Fig. 2.

Emulsified in both cases of HC dye and of basic dye, and when the amide alcohol is used (Examples 1 and 2) better dyeing properties were shown compared to when the surfactant is used (Comparative Examples 1 and 2).

### Experiment 3: Comparison of emulsifying property and smoothness due to differences in the blended amount of AMINACTOL

Emulsion type hair cosmetics were prepared according to the following formulations without using a dye, and the emulsifying property and smoothness were confirmed.

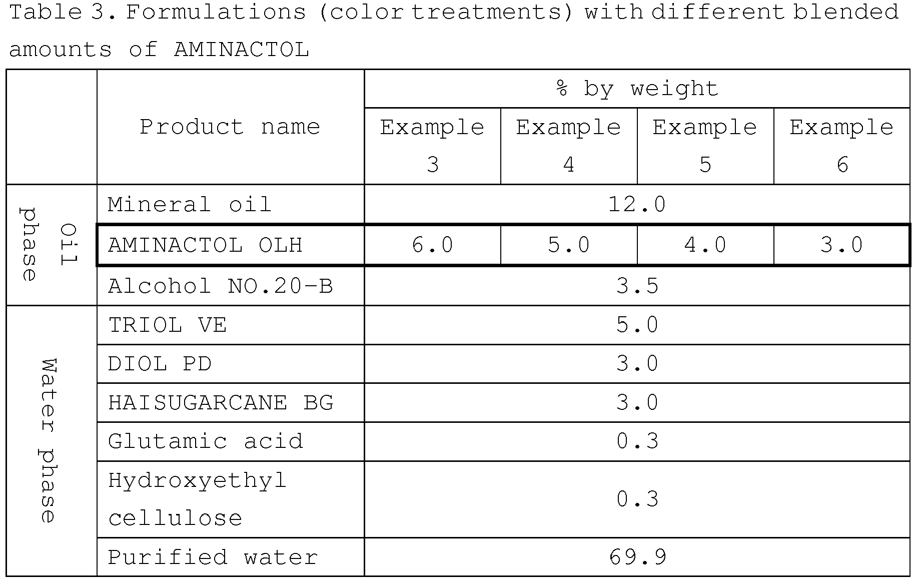

### Preparation method

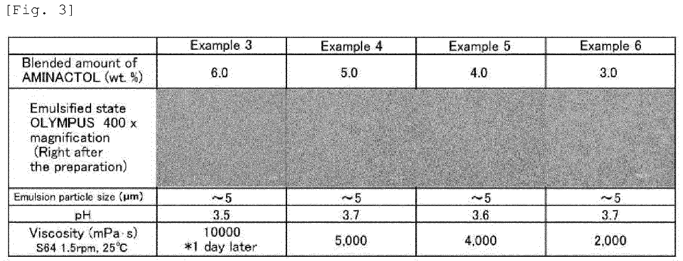

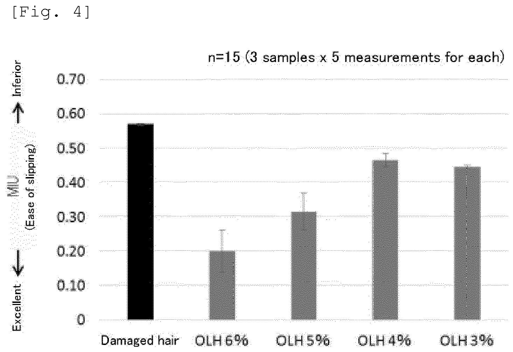

(1) Uniformly dissolve oil phase and water phase separately at 80°C
(2) Add oil phase while stirring water phase with an agitator (800rpm)
(3) After emulsification, stir with an agitator (2000rpm, 3min)
(4) Slowly cool to room temperature with manual stirring The evaluation results are shown in Fig. 3 and Fig. 4.

From the results in Fig. 3, it was confirmed that emulsification was sufficient with any blending amount.

The friction feeling test in Fig. 4 was performed using a friction feeling tester KES-SE (manufactured by Kato Tech Co., Ltd.) under the conditions of a sample stage temperature of 25 °C, a load of 25 g, a measurement speed of 1 mm/second, and five times of treatments.

From the results in Fig. 4, it was confirmed that the smoothness (ease of slipping) was improved when the blended amount of AMINACTOL was 5% by weight or more.

### Experiment 4: Comparison of emulsifying property and smoothness by different dyes (HC dye and basic dye)

Using HC Red No. 3 (manufactured by JOS.H.LOWENSTEIN & SONS, INC) and Basic Red 76 (manufactured by SENSIENT) as dyes, emulsion type hair cosmetics were prepared according to the following formulations.

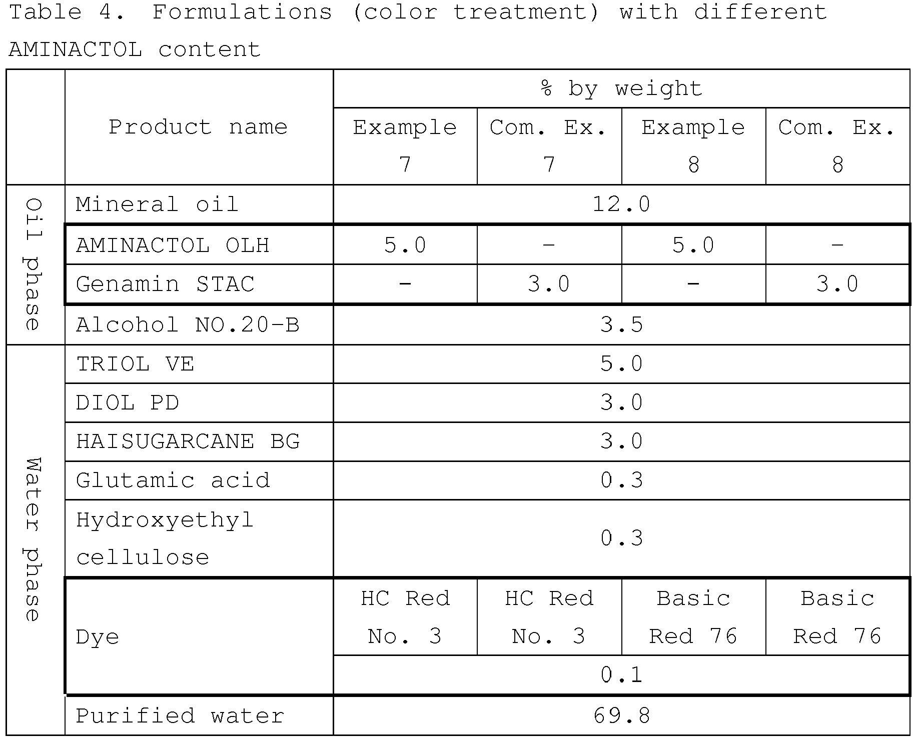

### Preparation method

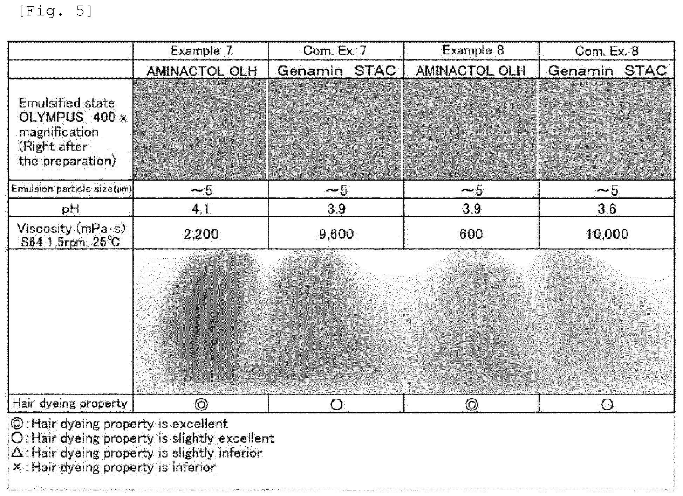

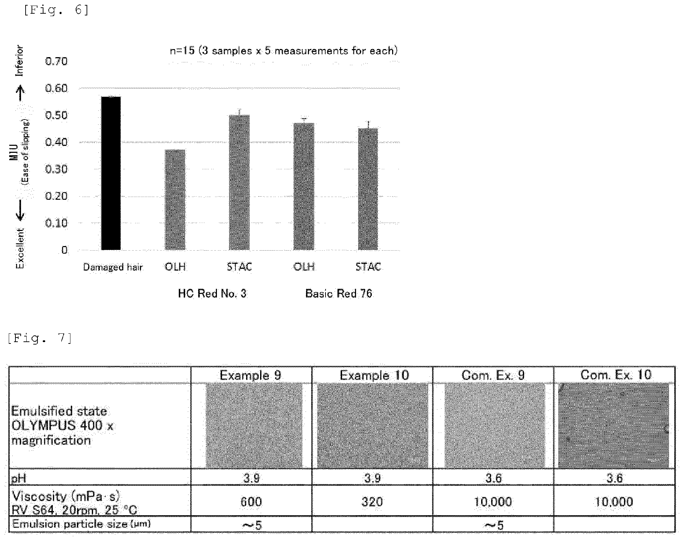

(1) Uniformly dissolve oil phase and water phase separately at 80°C
(2) Add oil phase while stirring water phase with an agitator (800rpm)
(3) After emulsification, stir with an agitator (2000rpm, 3min)
(4) Slowly cool to room temperature with manual stirring The evaluation results are shown in Fig. 5 and Fig. 6.

From the results in Fig. 5, it was confirmed that better hair dyeing properties were shown in both cases where HC dye was used and where basic dye was used, when using the amide alcohol compared with when using the surfactant.

The friction test in Fig. 6 was performed under the same conditions as in Fig. 4.

From the results in Fig. 6, it was confirmed that smoothness (ease of slipping) was improved in both cases where the HC dye was used and where the basic dye was used when using the amide alcohol compared to when using the surfactant.

### Experiment 5: Comparison of emulsified state and dyeing property with difference in dyes (basic dyes and acid dyes)

Each hair treatment was prepared in the same manner as in Experiments 1 to 4. Table 5 shows examples using basic dyes, and Table 6 shows examples using acid dyes.

Using basic red 76 (manufactured by SENSIENT) and basic blue 75 (manufactured by OsakaKaseihin co., Ltd.) as dyes, emulsion type hair cosmetics were prepared according to the following formulations.

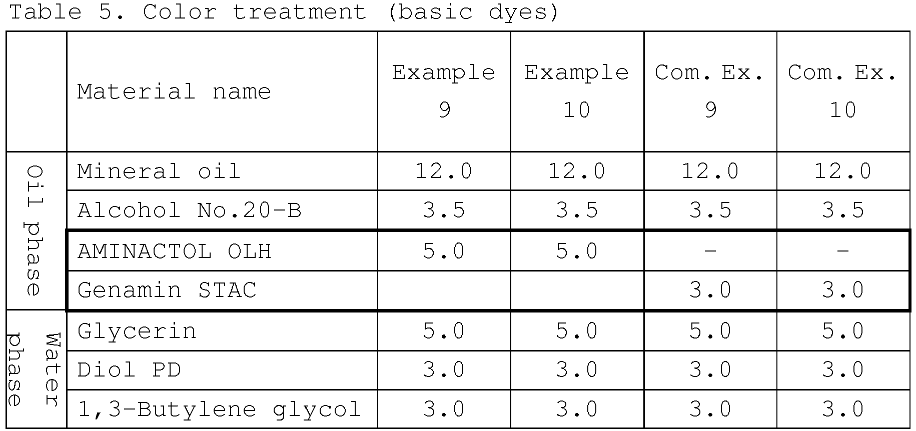

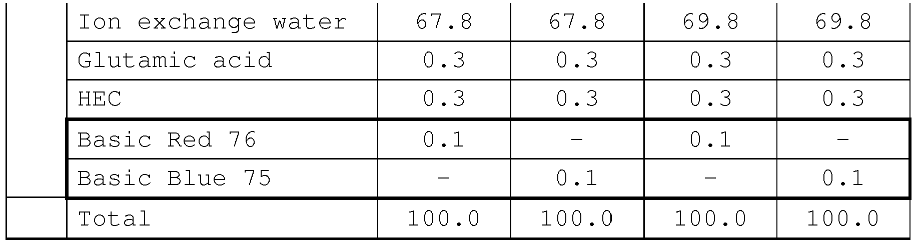

Using Black 401 (manufactured by Kishi Kasei Co., Ltd.) and Red 227 (manufactured by Kishi Kasei Co., Ltd.) as dyes, emulsion type hair cosmetics were prepared according to the following formulations.

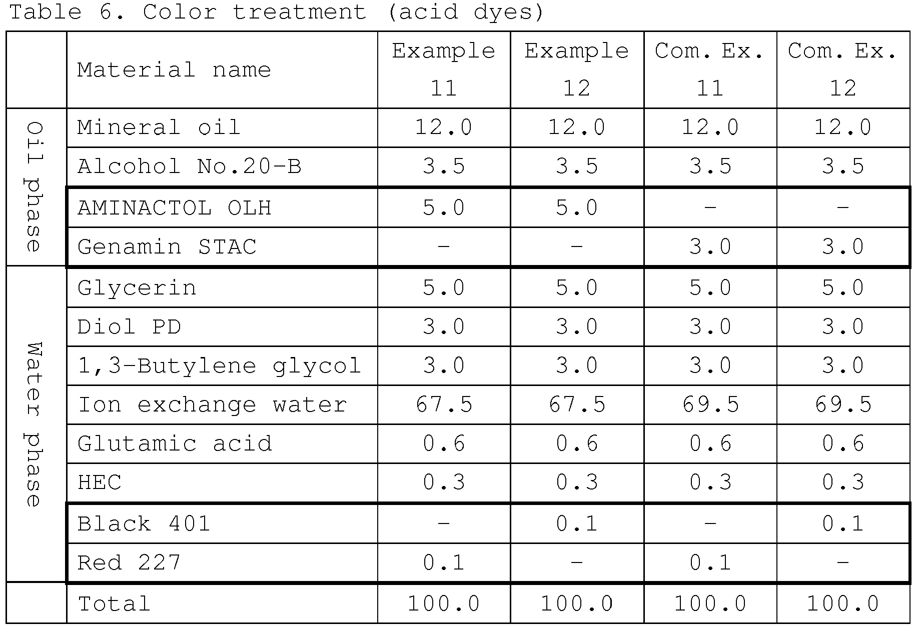

The results are shown in Fig. 7 to Fig. 9.

From the results in Fig. 7, it was confirmed that when the basic dye was used, good emulsifying property was exhibited.

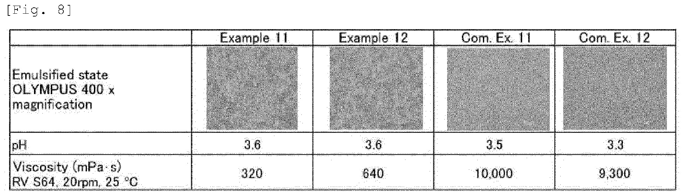

From the results in Fig. 8, it was confirmed that even when the acid dye was used, good emulsifying property was exhibited.

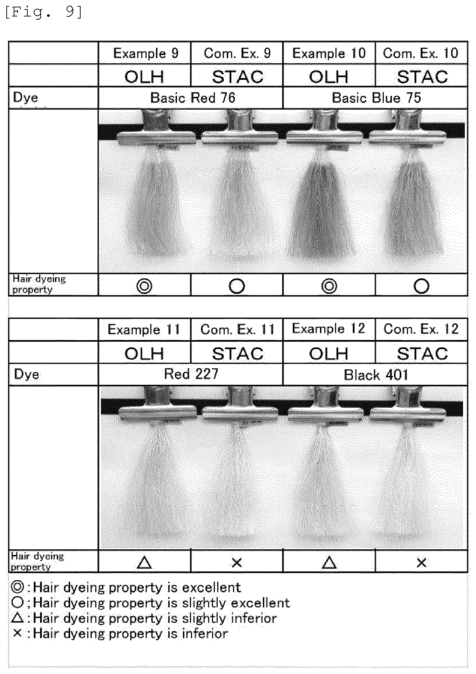

From the results in Fig. 9, it was confirmed that in both cases where basic dyes are used and where acid dyes are used, hair dyeing properties were improved with the use of the amide alcohol, compared with the use of the surfactant.

### Experiment 6: Analysis of the damaged hair treated with AMINACTOL OLH and Genamin STAC by TOF-SIMS

AMINACTOL OLH, Genamin STAC, the damaged hair, the hair treated with AMINACTOL OLH, and the hair treated with Genamin STAC were measured by time-of-flight secondary ion mass spectrometer method.

The measurement conditions are as follows:
Equipment: Time-of-flight secondary ion mass spectrometer (TRIFT III, manufactured by ULVAC-PHI, INCORPORATED.)
Primary ion source: Gallium ion (m/z69)
Primary ion output voltage: 15kV
Observation mass number range: 0.5-2000

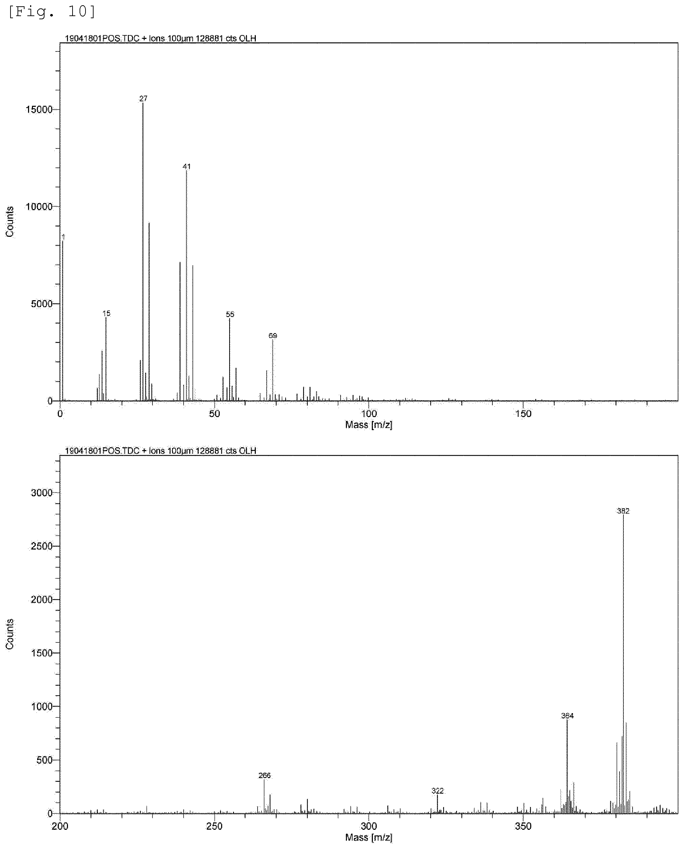

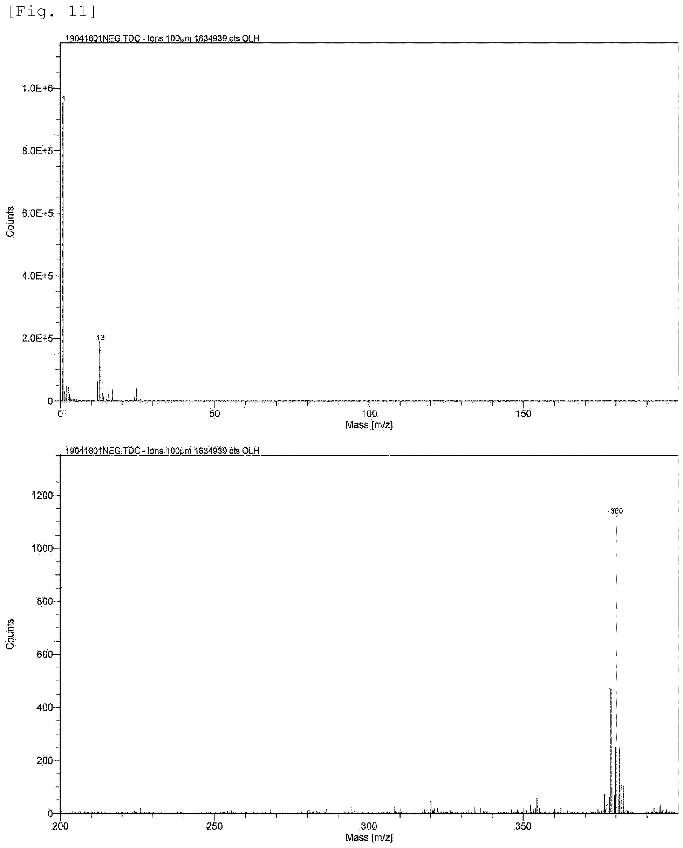

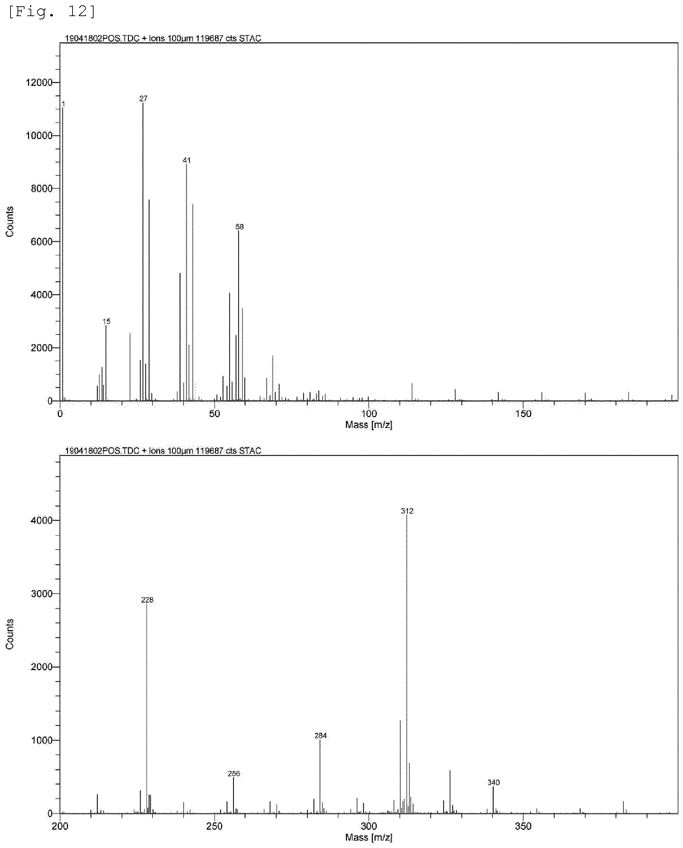

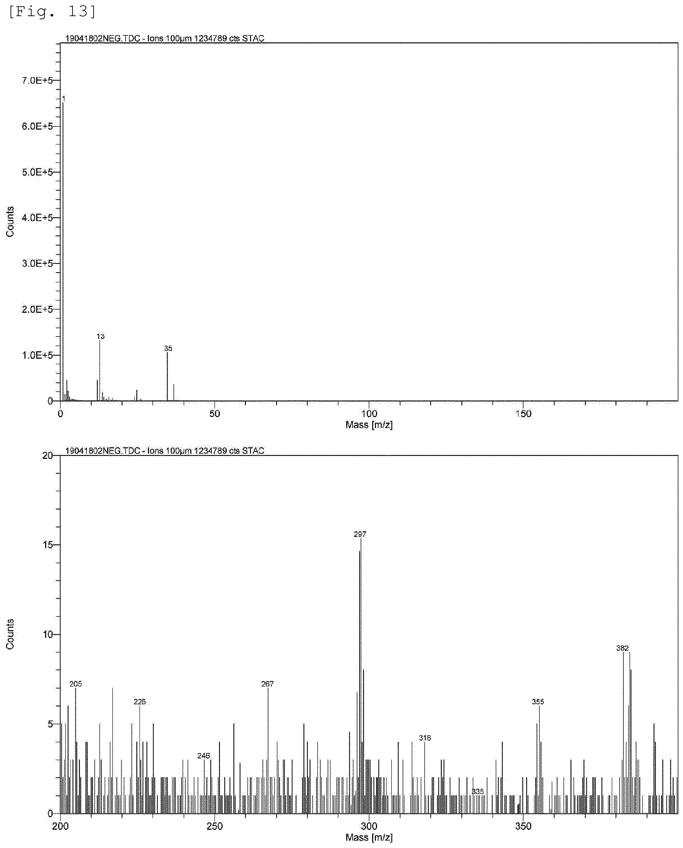

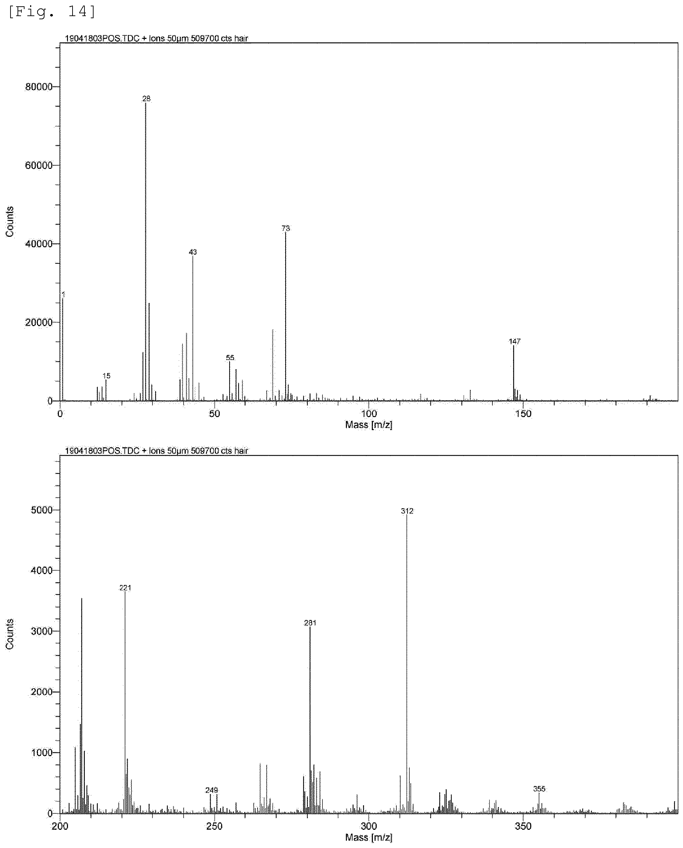

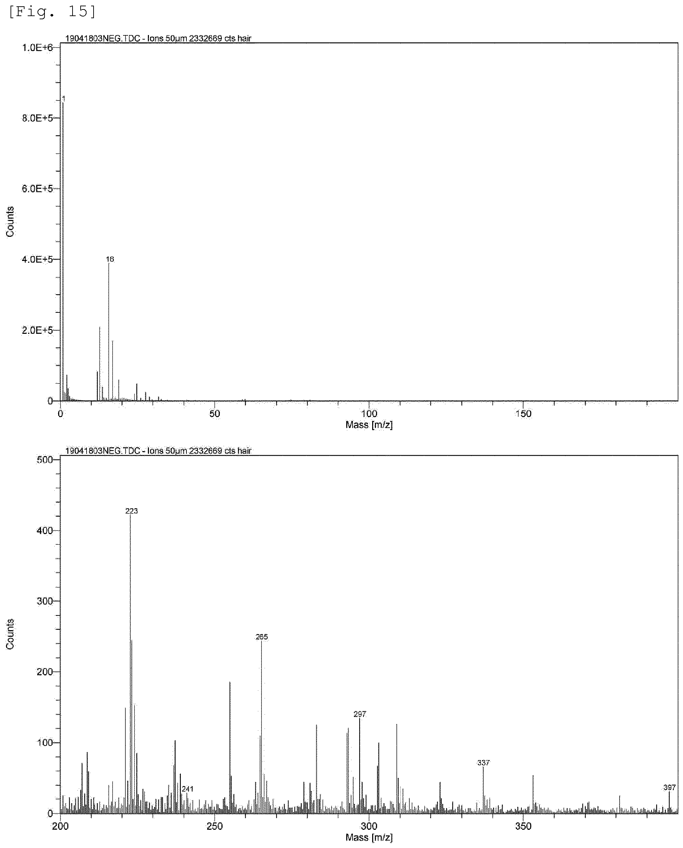

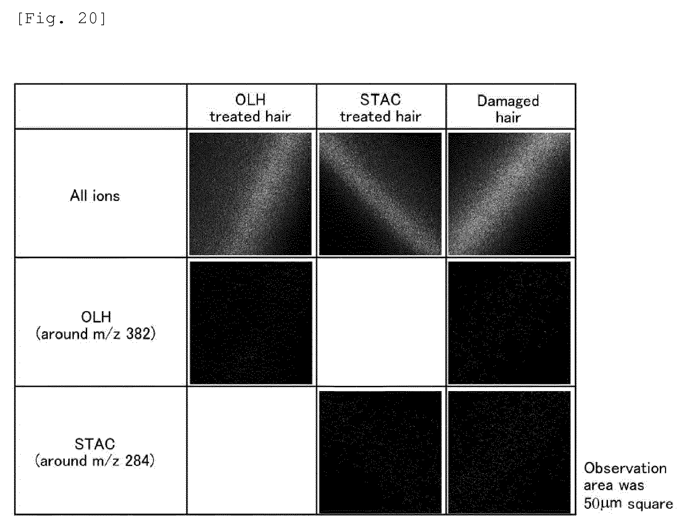

The measurement results (m / z 0 to 400) are shown in Fig. 10 to Fig.19, and the mapping image is shown in Fig. 20.

From the results in Fig. 10 to Fig. 15, the characteristic mass numbers of AMINACTOL OLH, Genamin STAC, and damaged hair were as follows.

**Table 7. Characteristic signals by TOF-SIMS**

| Sample name | Positive ion | Negative ion |
|---|---|---|
| AMINACTOL OLH | 266,322,364,382 | 380 |
| Genamin STAC | 228,256,284,312,326,340 | None |
| Damaged hair | 221,281,312 | 223,255,265,297 |

Note that since m/z 312 (positive ion) was detected from both "Genamin STAC" and "Damaged hair", it is excluded from the characteristic signals of "Genamin STAC".

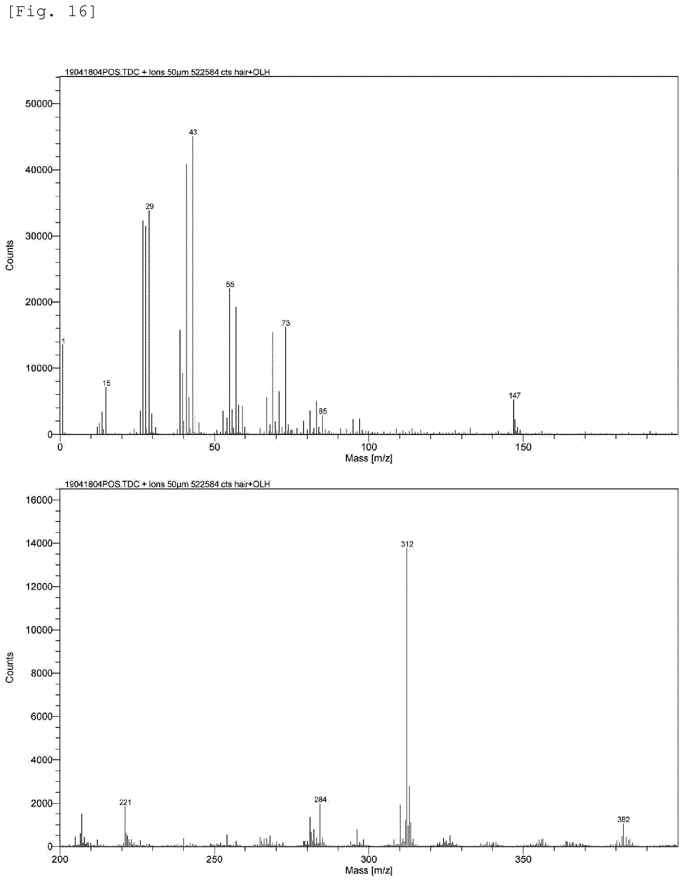

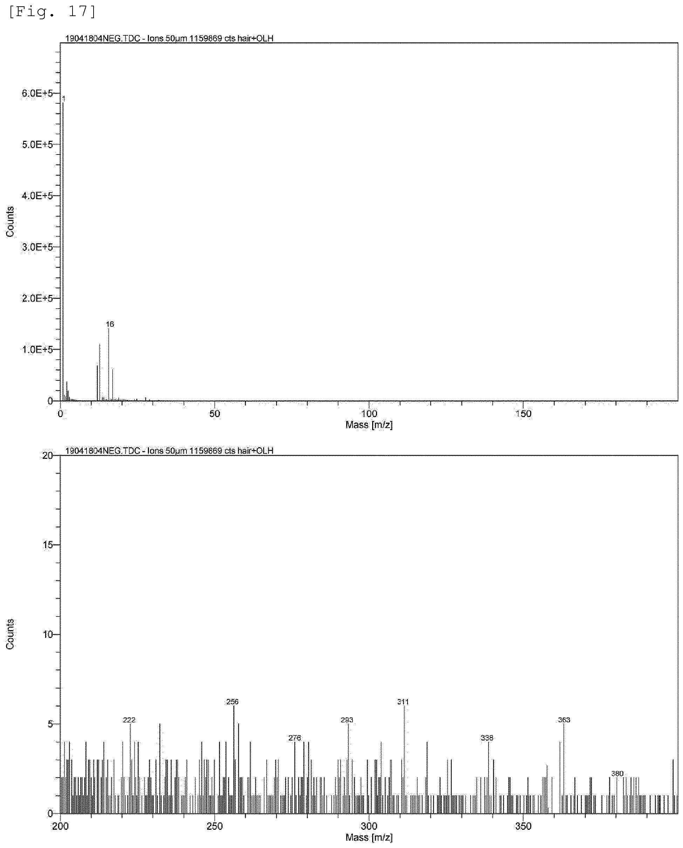

From the results in Fig. 16 and Fig. 17, a slight signal derived from "AMINACTOL OLH" was confirmed in (AMINACTOL) "OLH treated hair".

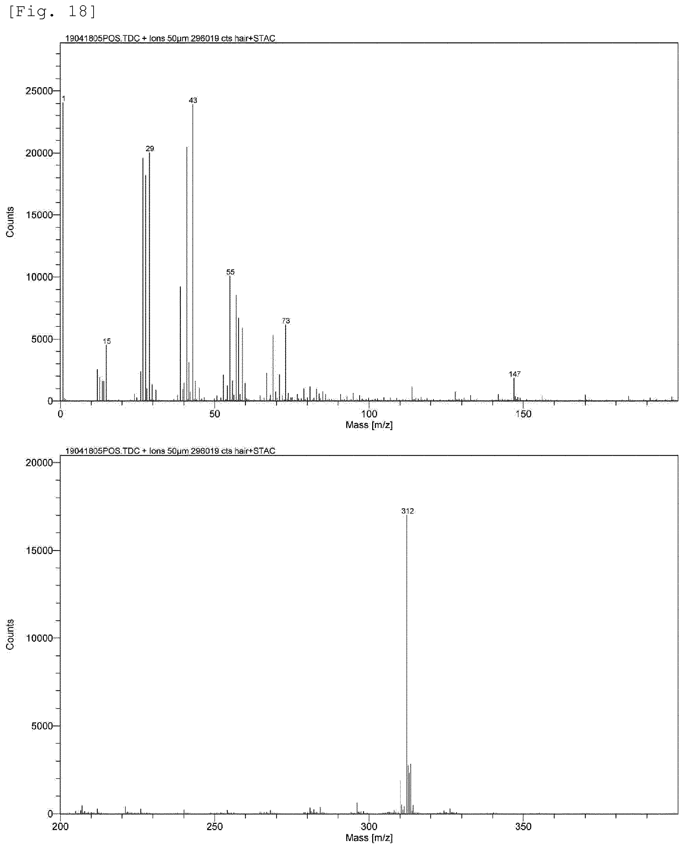

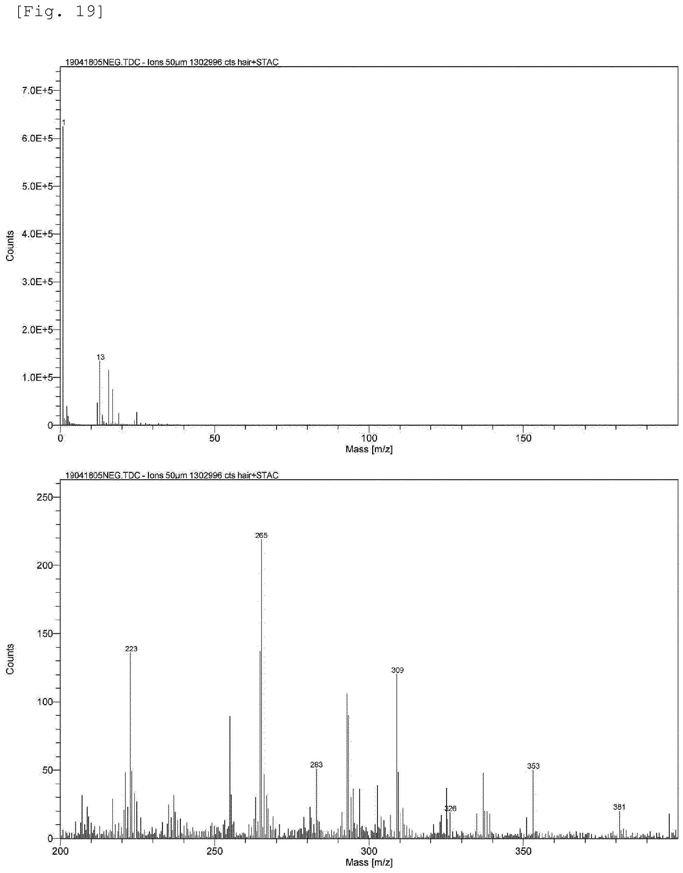

From the results in Fig. 18 and Fig. 19, no signal derived from "Genamin STAC" was detected in (Genamin) "STAC treated hair".

From this result, it is surmised that when AMINACTOL OLH is used, AMINACTOL OLH remains on the surface of the damaged hair, so that the hair dyeing property is improved as compared with the case where Genamin STAC is used.

The present invention has been described in detail above based on preferred embodiments, however, the present invention is not limited thereto, and each constituent can be replaced with any one that can exhibit the same function, or any constituent can be added.

### [Industrial Applicability]

The hair cosmetic of the present invention can be suitably used as a hair dyeing material such as a color treatment.

## Claims

1. A emulsion type hair cosmetic comprising an amide alcohol of formula (I) 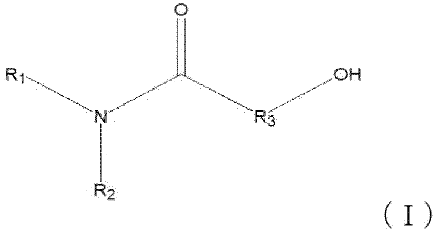 wherein
R₁ is an optionally substituted C6-C22 hydrocarbon group,
R₂ is H, or an optionally substituted C6-C22 hydrocarbon group,
R₃ is an optionally substituted, linear or branched C2-C21 hydrocarbon group,
an organic acid, and
any one dye selected from an HC dye, a basic dye and/or an acid dye.

2. The emulsion type hair cosmetic according to Claim 1 comprising the amide alcohol represented by formula (I) wherein R₁ is C10-C22 hydrocarbon group,
R₂ is H,
R₃ is C3-C12 hydrocarbon group.

3. The emulsion type hair cosmetic according to Claim 1 or 2, wherein the amide alcohol is

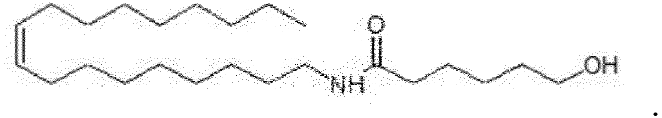

4. The emulsion type hair cosmetic according to any one of Claims 1 to 3 comprising
0.1 to 10.0% by mass of the amide alcohol represented by formula (I),
0.1 to 1.0% by mass of the organic acid, and
0.1 to 1.0% by mass of any one dye of an HC dye, a basic dye and/or acid dye.

5. The emulsion type hair cosmetic according to any one of Claims 1 to 4, wherein the molar ratio of the amide alcohol represented by formula (I) and the organic acid is 3:1 to 9:1.

6. The emulsion type hair cosmetic according to any one of Claims 1 to 5, wherein the emulsion type hair cosmetic is used for dyeing hair.

7. The emulsion type hair cosmetic according to any one of Claims 1 to 6, wherein the organic acid is one or more selected from the group consisting of monocarboxylic acid, dicarboxylic acid, hydroxycarboxylic acid, polycarboxylic acid, acidic amino acid and fatty acid.

8. The emulsion type hair cosmetic according to any one of Claims 1 to 7, wherein the organic acid is selected from glutamic acid, aspartic acid, lactic acid, glycolic acid, pyrrolidone carboxylic acid, malic acid, glycolic acid, formic acid, oxalic acid, benzoic acid, phosphoric acid, and sulfonic acid.

9. The emulsion type hair cosmetic according to any one of Claims 1 to 8 comprising one or more oil agents selected from the group consisting of an ester oil, a silicone oil, and a hydrocarbon oil.

10. The emulsion type hair cosmetic according to any one of Claims 1 to 9 which is substantially free of surfactants.

11. The emulsion type hair cosmetic according to any one of Claims 1 to 10 comprising glutamic acid as the organic acid.

12. The emulsion type hair cosmetic according to any one of Claims 9 to 11 comprising squalene, liquid paraffin and/or a non-volatile hydrocarbon oil as the oil agent.
